(19) Europäisches Patentamt
European Patent Office
Office européen des brevets

(11) EP 4 512 431 A2

(12) EUROPÄISCHE PATENTANMELDUNG

(43) Veröffentlichungstag:
26.02.2025 Patentblatt 2025/09

(21) Anmeldenummer: 24190222.0

(22) Anmeldetag: 23.07.2024

(51) Internationale Patentklassifikation (IPC):
**A61L 2/04** (2006.01)

(52) Gemeinsame Patentklassifikation (CPC):
**A61L 2/04;** A61L 2202/24

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: **25.07.2023 DE 102023119706**

(71) Anmelder: **B. Braun Avitum AG**
**34212 Melsungen (DE)**

(72) Erfinder:
• LIEB, Nino
34212 Melsungen (DE)
• ODERNHEIMER, Philipp
34212 Melsungen (DE)
• GIESA, Jonas
34121 Kassel (DE)

(74) Vertreter: **Winter, Brandl - Partnerschaft mbB**
**Alois-Steinecker-Straße 22**
**85354 Freising (DE)**

(54) **VERFAHREN ZUM DESINFIZIEREN EINES FLUIDLEITUNGSSYSTEMS EINER MEDIZINISCHEN VORRICHTUNG UND MEDIZINISCHE VORRICHTUNG**

(57) Die Offenbarung betrifft ein Verfahren zum Desinfizieren eines Fluidleitungssystems (4; 104) einer medizinischen Vorrichtung (2; 102). Offenbarungsgemäß werden bei einem Festlegen eines Soll-Verlaufs einer Fluidleitungssystemtemperatur ein Aufheizungskoeffizient, der ein zeitabhängiges Ansteigen der Fluidleitungssystemtemperatur bei einer Aufheizung des Fluidleitungssystems (4; 104) angibt, ein Passivabkühlungskoeffizient, der ein zeitabhängiges Absinken der Fluidleitungssystemtemperatur bei einer Passivabkühlung des Fluidleitungssystems (4; 104) angibt, und ein Einsatzbereitschaftszeitpunkt (t7), zu dem die Fluidleitungssystemtemperatur nach einem letztmaligen Unterschreiten einer Minimaldesinfektionstemperatur (T1) kleiner oder gleich einer maximalen Einsatzbereitschaftstemperatur (T3) sein muss, sodass die Vorrichtung (2; 102) nach dem Desinfizieren wieder einsatzbereit ist, berücksichtigt und erfolgt vor Erreichen eines vorbestimmten Minimalwerts der Soll-Letalität und vor dem letztmaligen Unterschreiten der Minimaldesinfektionstemperatur das Abkühlen des Fluidleitungssystems (4; 104) zumindest zeitweise mittels der Passivabkühlung durch alleiniges Beenden oder Unterbrechen des Heizens. Die Offenbarung betrifft des Weiteren eine Vorrichtung (2; 102), die ausgebildet ist, ein offenbarungsgemäßes Verfahren auszuführen.

Fig. 3

**Beschreibung**

Technisches Gebiet

Die vorliegende Offenbarung bezieht sich auf ein Verfahren zum Desinfizieren (d.h. zur Keimreduzierung um einen Faktor von mindestens $10^{-5}$) eines Fluidleitungssystems einer medizinischen Vorrichtung gemäß dem Oberbegriff des Anspruchs 1 sowie eine medizinische Vorrichtung gemäß dem Oberbegriff des Anspruchs 11.

Stand der Technik

Bei bekannten Vorrichtungen zur Desinfektion erfolgen Desinfektionsverfahren zeit- und temperaturgesteuert oder über eine AO-Wert Berechnung.

Bei zeit- und temperaturgesteuerten Desinfektionsverfahren wird ein Sollwert für eine Desinfektionstemperatur eingestellt. Nach Erreichen der Desinfektionstemperatur beginnt ein Timer eine Halte- und Sicherheitszeit aufzunehmen. Wenn über die gesamte Halte- und Sicherheitszeit die geforderte Desinfektionstemperatur gehalten wird, ist die Desinfektion erfolgreich. Danach erfolgt eine Abkühlphase.

Bei Desinfektionsverfahren mit A0-Wert-Berechnung wird ein Sollwert für den AO-Wert eingestellt. Nach Erreichen einer definierten Temperatur während der Aufheizphase werden Zeit und Temperaturwerte bei der AO-Wert Berechnung berücksichtigt. Wenn der AO-Wert erreicht ist, erfolgt die Abkühlphase.

Die bekannten Verfahren sind energetisch nicht optimal, weil mehr Energie dem System zugeführt wird, als für die Desinfektion benötigt wird. Temperatur und Zeitwerte bzw. der entsprechende AO-Wert werden während der Abkühlphase nicht berücksichtigt. Bei Geräten mit aktiver Kühlung erfolgt keine Bewertung des Zeitpunkts, wann eine geforderte Solltemperatur erreicht sein muss, wodurch ein erhöhter Bedarf an Kühlwasser benötigt wird.

Zusammenfassung der Offenbarung

Aufgabe der vorliegenden Erfindung ist es deshalb, eine Desinfektion eines Fluidleitungssystems einer medizinischen Vorrichtung zu ermöglichen, das hinsichtlich aufzubringender Wärme und/oder einzusetzender Menge an Desinfektionsflüssigkeit effizienter ist.

Diese Aufgabe wird durch eine Verfahren mit den Merkmalen des Anspruchs 1 sowie durch eine medizinische Vorrichtung mit den Merkmalen des Anspruchs 11 gelöst. Vorteilhafte Weiterbildungen sind Gegenstand der Unteransprüche.

Ein offenbarungsgemäßes Verfahren ist dafür ausgebildet, ein Fluidleitungssystem einer medizinischen Vorrichtung, insbesondere einer Blutbehandlungsvorrichtung, vorzugsweise einer Dialysemaschine, oder einer Umkehrosmoseanlage zu desinfizieren. "Desinfizieren" heißt insbesondere, eine Keimreduzierung um einen Faktor zwischen einschließlich $10^{-5}$ und ausschließlich $10^{-6}$ zu erreichen.

Das Verfahren weist folgende Schritte auf:

- Messen einer Fluidleitungssystemtemperatur in einem vorbestimmen Temperaturmessabschnitt innerhalb des Fluidleitungssystems, insbesondere mittels zumindest eines Temperatursensors, vorzugsweise mittels mehrerer über das Fluidleitungssystem verteilter Temperatursensoren, wobei für eine Überwachung und/oder Durchführung einer Desinfizierung bevorzugt ein Messwert desjenigen Temperatursensors als Fluidleitungssystemtemperatur herangezogen werden kann, der im Vergleich zu den übrigen Temperatursensoren die niedrigsten Temperaturen misst, und für eine Überwachung und/oder Durchführung einer Abkühlung bevorzugt ein Messwert desjenigen Temperatursensors als Fluidleitungssystemtemperatur herangezogen wird, der im Vergleich zu den übrigen Temperatursensoren die höchsten Temperaturen misst, oder wobei alternativ die Messwerte der Temperatursensoren beispielsweise gemittelt werden können,

- Festlegen eines Soll-Verlaufs der Fluidleitungssystemtemperatur mittels eines vorbestimmten Minimalwerts einer Soll-Letalität potentiell in dem Temperaturmessabschnitt vorhandener Keime, wobei die Soll-Letalität mittels Integration des Soll-Verlaufs der Fluidleitungssystemtemperatur über die Zeit ab einem erstmaligen Erreichen einer Minimaldesinfektionstemperatur, ab welcher potentiell in dem Temperaturmessabschnitt vorhandene Keime abgetötet werden können, bis zu einem letztmaligen Unterschreiten der Minimaldesinfektionstemperatur berechnet wird,

- Heizen des Fluidleitungssystems mittels eines Desinfektionsfluids in (Zeit- bzw. Heizzeit-)Abschnitten des Soll-Verlaufs der Fluidleitungssystemtemperatur, in welchen die Fluidleitungssystemtemperatur erhöht oder konstant gehalten werden soll, und

- Abkühlen des Fluidleitungssystems in (Zeit- bzw. Abkühlzeit-)Abschnitten des Soll-Verlaufs der Fluidleitungssystemtemperatur, in welchen die Fluidleitungssystemtemperatur gesenkt werden soll.

Eine Ist-Letalität wird insbesondere entsprechend der Soll-Letalität berechnet. Das heißt, ein Ist-Verlauf der Fluidleitungssystemtemperatur wird über die Zeit ab dem erstmaligen Erreichen der Minimaldesinfektionstemperatur bis zu dem letztmaligen Unterschreiten der Minimaldesinfektionstemperatur integriert. Bei der Berechnung der Soll-Letalität und entsprechend bei der Berechnung der Ist-Letalität werden insbesondere Zeitabschnitte nicht berücksichtigt bzw. nicht integriert, in welchen die gemessene Fluidleitungssystemtemperatur kleiner der Minimaldesinfektionstemperatur ist.

Offenbarungsgemäß werden bei dem Festlegen des Soll-Verlaufs der Fluidleitungssystemperatur ein Aufheizungskoeffizient, ein Passivabkühlungskoeffizient und ein Einsatzbereitschaftszeitpunkt berücksichtigt.

Der Aufheizungskoeffizient gibt ein zeitabhängiges Ansteigen der Fluidleitungssystemtemperatur bei einer Aufheizung des Fluidleitungssystems an. Der Passivabkühlungskoeffizient gibt ein zeitabhängiges Absinken der Fluidleitungssystemtemperatur bei einer Passivabkühlung des Fluidleitungssystems an. Der Einsatzbereitschaftszeitpunkt ist ein Zeitpunkt, zu dem die Fluidleitungssystemtemperatur nach dem letztmaligen Unterschreiten der Minimaldesinfektionstemperatur kleiner oder gleich einer maximalen Einsatzbereitschaftstemperatur sein muss, sodass die Vorrichtung nach dem Desinfizieren wieder einsatzbereit ist.

Offenbarungsgemäß erfolgt vor Erreichen des vorbestimmten Minimalwerts der Soll-Letalität bzw. bevor ein Wert der Ist-Letalität dem vorbestimmten Minimalwert der Soll-Letalität entspricht und vor dem letztmaligen Unterschreiten der Minimaldesinfektionstemperatur, das Abkühlen des Fluidleitungssystems zumindest zeitweise mittels der Passivabkühlung durch alleiniges Beenden oder Unterbrechen des Heizens erfolgt.

Dadurch, dass bei der Berechnung der Ist-Letalität auch Zeitabschnitte nach einem Beenden oder Unterbrechen des Heizens bedacht werden, kann eine Einsatzzeit eines Heizers bzw. einer Heizeinrichtung und eine Dauer eines Desinfektionsverfahrens bei gleichbleibendem vorbestimmten Minimalwert der Soll-Letalität verkürzt werden.

Gemäß einem Aspekt der Offenbarung können/kann der Aufheizungskoeffizient und/oder der Passivabkühlungskoeffizient vor dem Festlegen des Soll-Verlaufs der Fluidleitungssystemtemperatur, insbesondere vor oder nach dem Messen der Fluidleitungssystemtemperatur in dem vorbestimmen Temperaturmessabschnitt, mittels zumindest eines Versuchs und/oder mittels einer Simulation festgelegt werden. Der Aufheizungskoeffizient kann insbesondere während bzw. zu Beginn des Verfahrens während eines Betriebs eines Heizers anhand von Messdaten erkannt bzw. ermittelt werden. Der Passivabkühlungskoeffizient kann insbesondere während bzw. zu Beginn des Verfahrens durch zeitweises Ausschaltens des Heizers anhand von Messdaten erkannt bzw. ermittelt werden. Der Aufheizungskoeffizient und/oder der Passivabkühlungskoeffizient können/kann insbesondere mittels miteinander vernetzter Geräte schrittweise angepasst werden, welche Messdaten über Cloud- und/oder Edge-Computing analysieren. Der Aufheizungskoeffizient und/oder der Passivabkühlungskoeffizient können/kann insbesondere durch thermodynamische Gleichungen, vorzugsweise iterativ, mittels einer offenbarungsgemäßen Steuerungseinrichtung bzw. einer Berechnungseinrichtung errechnet werden. Die Simulation oder ein simuliertes Model des Fluidleitungssystems für die Ermittlung des Aufheizungskoeffizienten und/oder des Passivabkühlungskoeffizienten kann von der offenbarungsgemäßen Steuerungseinrichtung bzw. der Berechnungseinrichtung insbesondere auf der Grundlage von Flüssigkeitsdaten (beispielsweise einer spezifischen Wärmekapazität, eines Gesamtvolumens), einer Rohrleitungsgeometrie (Länge, Durchmesser und/oder, Materialstärke), einer Dämmung (der Rohrleitungen), eines oder mehrerer Wärmeübertragungskoeffizienten des Rohrleitungs-/Anlagenmaterials, einer Außentemperatur und/oder einer effektiven Heizleistung in dem Fluidleitungssystem erstellt werden.

Werden/wird der Aufheizungskoeffizient und/oder der Passivabkühlungskoeffizient vor dem Festlegen des Soll-Verlaufs der Fluidleitungssystemtemperatur festgelegt, können Aufheiz- und/oder Abkühlphasen in vorteilhafter Weise bei der Desinfektion bedacht werden.

Gemäß einem Aspekt der Offenbarung können/kann der Aufheizungskoeffizient und/oder der Passivabkühlungskoeffizient während einer Durchführung des Verfahrens überwacht werden und kann bei einer Änderung des Aufheizungskoeffizienten und/oder des Passivabkühlungskoeffizienten der Schritt des Festlegens des Soll-Verlaufs der Fluidleitungssystemtemperatur wiederholt ausgeführt werden. Anders ausgedrückt, können/kann der Aufheizungskoeffizient und/oder der Passivabkühlungskoeffizient während einer Durchführung des Verfahrens anhand von Messdaten neu berechnet werden.

Werden/wird der Aufheizungskoeffizient und/oder der Passivabkühlungskoeffizient überwacht und gegebenenfalls angepasst, kann die Genauigkeit bei der Berechnung der Ist-Letalität verbessert werden.

Gemäß einem Aspekt der Offenbarung kann bei dem Festlegen des Soll-Verlaufs der Fluidleitungssystemtemperatur ein Aktivabkühlungskoeffizient berücksichtigt werden, der ein zeitabhängiges Absinken der Fluidleitungssystemtemperatur bei einer Aktivabkühlung des Fluidleitungssystems angibt, und kann das Abkühlen des Fluidleitungssystems zeitweise mittels der Aktivabkühlung durch Abführen warmen Desinfektionsfluids und/oder Zuführen eines Kühlmittels erfolgen. Das Desinfektionsfluid kann insbesondere mit Chemikalien versetzt sein. Das Kühlmittel kann insbesondere von der Art eines Fluids, beispielsweise eines Prozesswassers, sein, welches während eines regulären Betriebs der medizinischen Vorrichtung verwendet wird. Der Aktivabkühlungskoeffizient kann insbesondere durch thermodynamische Gleichungen, vorzugsweise iterativ, mittels der offenbarungsgemäßen Steuerungseinrichtung bzw. der Berechnungseinrichtung errechnet werden. Die Simulation oder ein simuliertes Model des Fluidleitungssystems für die Ermittlung des Aktivabkühlungskoeffizienten kann von der offenbarungsgemäßen Steuerungseinrichtung bzw. der Berechnungseinrichtung insbesondere auf der Grundlage von Flüssigkeitsdaten (beispielsweise einer spezifischen Wärmekapazität, eines Gesamtvolumens), einer Rohrleitungsgeometrie (Länge, Durchmesser und/oder, Materialstärke), einer Dämmung (der Rohrleitungen), eines oder mehrerer Wärmeübertragungskoeffizienten des Rohrleitungs-/Anlagenmaterials, einer Außentemperatur und/oder einer effektiven Heizleistung in dem Fluidleitungssystem erstellt werden.

Wird bei dem Festlegen des Soll-Verlaufs ein Aktivkühlungskoeffizient bedacht, können Phasen, in welchen das Fluidleitungssystem aktiv gekühlt wird, in vorteilhafter Weise bei der Desinfektion bedacht werden.

Gemäß einem Aspekt der Offenbarung kann der Aktivabkühlungskoeffizient vor dem Festlegen des Soll-Verlaufs der Fluidleitungssystemtemperatur, insbesondere vor oder nach dem Messen der Fluidleitungssystemtemperatur in dem vorbestimmten Temperaturmessabschnitt, mittels zumindest eines Versuchs und/oder mittels einer Simulation festgelegt werden. Der Aktivabkühlungskoeffizient kann insbesondere während bzw. zu Beginn des Verfahrens durch zeitweises Ausschalten des Heizers und gleichzeitiges Abführen warmen Desinfektionsfluids und/oder Zuführen eines Kühlmittels anhand von Messdaten erkannt bzw. ermittelt werden.

Wird der Aufheizungskoeffizient vor dem Festlegen des Soll-Verlaufs der Fluidleitungssystemtemperatur festgelegt, können Aktivabkühlphasen in vorteilhafter Weise bei der Desinfektion bedacht werden.

Gemäß einem Aspekt der Offenbarung kann der Aktivabkühlungskoeffizient während einer Durchführung des Verfahrens überwacht werden und kann bei einer Änderung des Aktivabkühlungskoeffizienten der Schritt des Festlegens des Soll-Verlaufs der Fluidleitungssystemtemperatur wiederholt ausgeführt werden. Anders ausgedrückt, kann der Aktivabkühlungskoeffizient während einer Durchführung des Verfahrens anhand von Messdaten neu berechnet werden.

Wird der Aktivabkühlungskoeffizient überwacht und gegebenenfalls angepasst, kann die Genauigkeit bei dem Festlegen des Soll-Verlaufs hinsichtlich des Erreichens des Einsatzbereitschaftszeitpunkts verbessert werden.

Gemäß einem Aspekt der Offenbarung kann mittels der vorbestimmten Soll-Letalität, des Einsatzbereitschaftszeitpunkts, des Passivabkühlungskoeffizienten und des Aktivabkühlungskoeffizienten ein letztmöglicher Aktivabkühlungsstartzeitpunkt berechnet werden, nach welchem die maximale Einsatzbereitschaftstemperatur nur noch mittels der Aktivkühlung erreicht werden kann und bis zu welchem das Fluidleitungssystem ausschließlich mittels der Passivkühlung gekühlt wird. Die Passivkühlung kann insbesondere zum Großteil durch freie Konvektion erfolgen.

Gemäß einem Aspekt der Offenbarung kann bei einer Festlegung des vorbestimmten Minimalwerts der Soll-Letalität ein Sicherheitsfaktor einberechnet werden.

Wird der vorbestimmte Minimalwert der Soll-Letalität größer festgelegt als theoretisch notwendig, kann eine Wahrscheinlichkeit für eine unbeabsichtigt unzureichende Desinfektion verringert werden.

Gemäß einem Aspekt der Offenbarung kann der Soll-Verlauf der Fluidleitungssystemtemperatur derart festgelegt werden, dass ein mittleres Niveau der Fluidleitungssystemtemperatur zwischen dem erstmaligen Erreichen der Minimaldesinfektionstemperatur und dem letztmaligen Unterschreiten der Minimaldesinfektionstemperatur über der Minimaldesinfektionstemperatur liegt.

Wird das mittlere Niveau der Fluidleitungssystemtemperatur oberhalb der Minimaldesinfektionstemperatur festgelegt, kann eine Wahrscheinlichkeit für eine unbeabsichtigt unzureichende Erwärmung verringert werden.

Gemäß einem Aspekt der Offenbarung kann das mittlere Niveau der Fluidleitungssystemtemperatur zwischen dem erstmaligen Erreichen der Minimaldesinfektionstemperatur und dem letztmaligen Unterschreiten der Minimaldesinfektionstemperatur in Richtung der Minimaldesinfektionstemperatur abgesenkt werden, wenn erkannt wird, dass die Fluidleitungssystemtemperatur nach dem letztmaligen Unterschreiten der Minimaldesinfektionstemperatur ohne der Aktivkühlung vor dem Einsatzbereitschaftszeitpunkt auf die maximale Einsatzbereitschaftstemperatur abgesenkt werden kann.

Wird das mittlere Niveau der Fluidleitungssystemtemperatur bedarfsweise in Richtung der Minimaldesinfektionstemperatur abgesenkt, kann die Ressourceneffizienz der Desinfektion gesteigert werden.

Die Offenbarung betrifft des Weiteren eine medizinische Vorrichtung, insbesondere eine Blutbehandlungsvorrichtung, vorzugsweise eine Dialysemaschine, oder eine Umkehrosmoseanlage, mit einem Fluidleitungssystem, zumindest einem Temperatursensor, der ausgebildet ist, eine Fluidleitungssystemtemperatur in einem vorbestimmen Temperaturmessabschnitt innerhalb des Fluidleitungssystems messen zu können, einem Einlass zum Einleiten eines Desinfektionsfluids und/oder eines Kühlmittels in das Fluidleitungssystem, einem Auslass zum Ausleiten des Desinfektionsfluids und/oder des Kühlmittels aus dem Fluidleitungssystem, einer Heizeinrichtung zum Heizen des Desinfektionsfluids und einer Steuerungseinrichtung zum Steuern des Einlasses, des Auslasses und der Heizeinrichtung in Abhängigkeit der von dem zumindest einen Temperatursensor gemessenen Fluidleitungssystemtemperatur

Offenbarungsgemäß ist die Steuerungseinrichtung ausgebildet, in Reaktion auf eine Anforderung ein offenbarungsgemäßes Verfahren auszuführen.

Kurzbeschreibung der Zeichnungen

Die vorliegende Offenbarung wird im Folgenden anhand bevorzugter Ausführungsbeispiele unter Bezugnahme auf die beigefügten Zeichnungen näher beschrieben. Es zeigen:

Fig. 1 eine schematische Ansicht einer offenbarungsgemäßen medizinischen Vorrichtung gemäß einer ersten Ausführungsform;

Fig. 2 eine schematische Ansicht einer medizinischen Vorrichtung gemäß einer zweiten Ausführungsform;

Fig. 3 ein schematisches Temperatur-Zeit-Diagramm eines offenbarungsgemäßen Verfahrens;

Fig. 4 ein Diagramm, in welchem die Temperatur und der AO-Wert über die Zeit während eines offenbarungsgemäßen Verfahrens bis zum Erreichen eines vorbestimmten Minimalwerts einer Letalität gezeigt ist;

Fig. 5 ein der Fig. 4 entsprechendes Diagramm zu einem offenbarungsgemäßen Verfahren, bei dem ein erhöhter vorbestimmter Minimalwert der Letalität erreicht wird; und

Fig. 6 Diagramme, in welchen die Temperatur, der AO-Wert und ein Systemzustand einer medizinischen Vorrichtung während eines offenbarungsgemäßen Verfahrens gezeigt sind.

Detaillierte Beschreibung bevorzugter Ausführungsformen

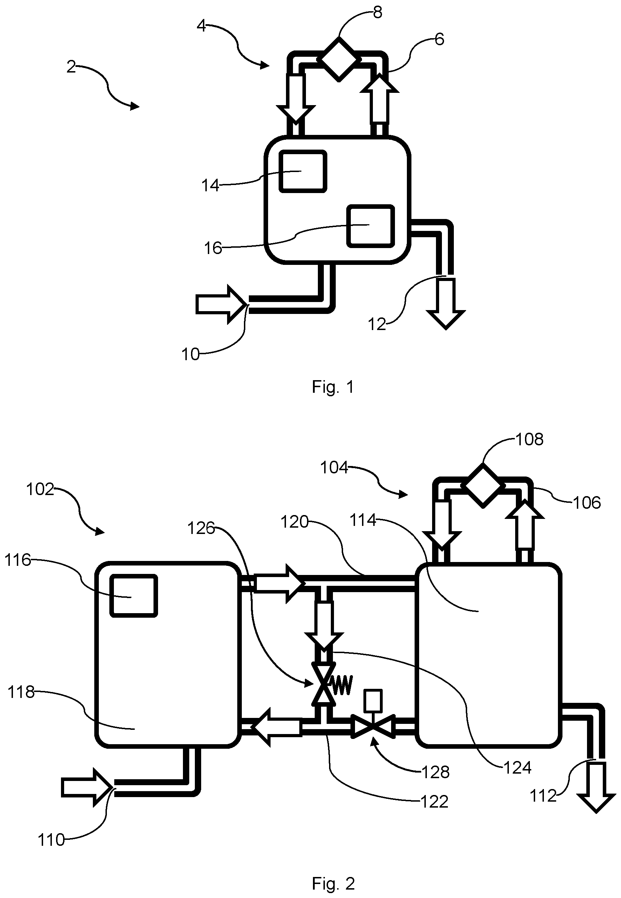

Fig. 1 zeigt eine schematische Ansicht einer offenbarungsgemäßen medizinischen Vorrichtung 2 gemäß einer ersten Ausführungsform. Die Vorrichtung ist als eine Umkehrosmoseanlage ausgebildet und weist ein Fluidleitungssystem 4 auf. Das Fluidleitungssystem 4 ist mit einer Ringleitung 6 ausgestattet, an welcher ein Temperatursensor 8 vorgesehen ist. Der Temperatursensor 8 ist ausgebildet, eine offenbarungsgemäße Fluidleitungssystemtemperatur messen zu können. Alternativ könnten mehrere über das Fluidleitungssystem 4 verteilte Temperatursensoren vorgesehen sein, wobei für eine Überwachung und/oder Durchführung einer Desinfizierung ein Messwert desjenigen Temperatursensors als Fluidleitungssystemtemperatur herangezogen wird, der im Vergleich zu den übrigen Temperatursensoren die niedrigsten Temperaturen misst, und für eine Überwachung und/oder Durchführung einer Abkühlung ein Messwert desjenigen Temperatursensors als Fluidleitungssystemtemperatur herangezogen wird, der im Vergleich zu den übrigen Temperatursensoren die höchsten Temperaturen misst. Zusätzlich dazu können Temperatursensoren in der medizinischen Vorrichtung 2, insbesondere abseits des Fluidleitungssystems 4, vorhanden sein, welche in gleicher Weise zur Berechnung mit herangezogen werden können, insbesondere wenn nicht auszuschließen ist, dass sich die wärmste bzw. kälteste Stelle außerhalb des Fluidleitungssystems 4 befindet.

Die Vorrichtung 2 weist einen Einlass 10 auf, über welchen ein Desinfektionsfluid und/oder Kühlwas

ser in das Fluidleitungssystem 4 in die Vorrichtung 2 eingeleitet werden kann.

Die Vorrichtung 2 weist des Weiteren einen Auslass 12 auf, über welchen Desinfektionsfluid und/oder Kühlmittel aus der Vorrichtung 2 ausgeleitet werden kann.

Die Vorrichtung 2 ist mit einer Heizeinrichtung 14 ausgestattet, die ausgebildet ist, in der Vorrichtung 2 bzw. in dem Fluidleitungssystem 4 befindliches Desinfektionsfluid aufwärmen bzw. erhitzen zu können.

Die Vorrichtung 2 weist eine Steuerungseinrichtung 16 auf, die ausgebildet ist, den Einlass 10, den Auslass 12 und/oder die Heizeinrichtung 14 parallel oder seriell steuern zu können.

Um die Vorrichtung 2 bzw. das Fluidleitungssystem 4 zu desinfizieren, wird über den Einlass 10 Prozesswasser eingeleitet, welches in der Vorrichtung 2 mittels der Heizeinrichtung 14 erwärmt wird. Das erwärmte Wasser wird als Desinfektionsfluid in dem Fluidleitungssystem 4 bzw. in der Ringleitung 6 verteilt. Um Fluid in der Vorrichtung 2 bzw. in dem Fluidleitungssystem 4 zu fördern, kann die Vorrichtung 2 eine (nicht gezeigte) Pumpe aufweisen.

Die Desinfektion wird von der Steuerungseinrichtung 16 mittels Berechnung einer Letalität potentiell in dem Fluidleitungssystem 4 bzw. in der Ringleitung 6 vorhandener Keime gesteuert.

Als Kennzahl für die Letalität wird der AO-Wert herangezogen, der gemäß ISO 15883 durch nachfolgende Formel berechnet wird:

$$A_0 = \sum 10^{\frac{[T(t)-80°C]}{z}} \cdot \Delta t$$

"z" ist der z-Wert, für dessen Definition beispielsweise auf die ISO 11139 zurückgegriffen werden kann. Für den AO-Wert gilt "z = 10°C".

"T(t)" ist die Temperatur des Desinfektionsfluids in °C in Abhängigkeit der Zeit "t".

"$\Delta t$" ist das gewählte Zeitintervall in Sekunden.

Sobald ein vorbestimmter Minimalwert des AO-Wert erreicht wurde, beendet die Vorrichtung 2 bzw. die Steuerungseinrichtung 16 die thermische Desinfektion und wechselt in eine Abkühlphase, indem warmes Wasser aus dem Auslass 12 geleitet und durch kälteres Prozesseingangswasser ersetzt wird.

Fig. 2 zeigt eine schematische Ansicht einer medizinischen Vorrichtung 102 gemäß einer zweiten Ausführungsform. Die Vorrichtung 102 gemäß der zweiten Ausführungsform unterscheidet sich von der Vorrichtung 2 gemäß der ersten Ausführungsform derart, dass bei der letztgenannten die Heizeinrichtung 14 integral ausgebildet ist, wohingegen eine Heizeinrichtung 114 gemäß der zweiten Ausführungsform separat von einer Umkehrosmoseeinrichtung 118 ausgebildet ist. Die Heizeinrichtung 114 ist mit der Umkehrosmoseeinrichtung 118 über eine Kühlfluidleitung 120 und eine Warmfluidleitung 122 verbunden.

Über die Kühlfluidleitung 120 kann Fluid von der Umkehrosmoseeinrichtung 118 zu der Heizeinrichtung 114 geleitet werden. Über die Warmfluidleitung 122 kann Fluid von der der Heizeinrichtung 114 zu der Umkehrosmoseeinrichtung 118 geleitet werden. Die Kühlfluidleitung 120 und die Warmfluidleitung 122 sind miteinander über einer Rückführleitung 124 verbunden, die seitlich von einem Abschnitt der Kühlfluidleitung 120 abzweigt und seitlich in einen Abschnitt der Warmfluidleitung 122 einzweigt. In der Rückführleitung 124 ist ein Überströmventil 126 vorgesehen. In der Warmfluidleitung 122 ist stromauf der Einzweigung der Rückführleitung 124 ein Schaltventil 128 vorgesehen.

Entsprechend der Vorrichtung 2 gemäß der ersten Ausführungsform weist die Vorrichtung 102 gemäß der zweiten Ausführungsform ein Fluidleitungssystem 104 mit einer Ringleitung 106 auf. Die Ringleitung 106 ist mit einem Temperatursensor 108 versehen, um die Fluidleitungssystemtemperatur eines in dem Fluidleitungssystem 104 bzw. in der Ringleitung 106 befindlichen Fluids messen zu können. Alternativ könnten mehrere über das Fluidleitungssystem 104 verteilte Temperatursensoren vorgesehen sein, wobei für eine Überwachung und/oder Durchführung einer Desinfizierung ein Messwert desjenigen Temperatursensors als Fluidleitungssystemtemperatur herangezogen wird, der im Vergleich zu den übrigen Temperatursensoren die niedrigsten Temperaturen misst, und für eine Überwachung und/oder Durchführung einer Abkühlung ein Messwert desjenigen Temperatursensors als Fluidleitungssystemtemperatur herangezogen wird, der im Vergleich zu den übrigen Temperatursensoren die höchsten Temperaturen misst.

Die Vorrichtung 102 weist einen Einlass 110 an der Umkehrosmoseeinrichtung 118 auf, über welchen ein Desinfektionsfluid und/oder Kühlwasser in das Fluidleitungssystem 104 in die Vorrichtung 102 eingeleitet werden kann.

Die Vorrichtung 102 weist des Weiteren einen Auslass 112 an der Heizeinrichtung 114 auf, über welchen Desinfektionsfluid und/oder Kühlmittel aus der Vorrichtung 102 ausgeleitet werden kann.

Wie die integrale Heizeinrichtung 14 gemäß der ersten Ausführungsform ist die separat ausgebildete Heizeinrichtung 114 gemäß der zweiten Ausführungsform ausgebildet, in der Vorrichtung 102 bzw. in dem Fluidleitungssystem 104 befindliches Desinfektionsfluid aufwärmen bzw. erhitzen zu können.

Die Vorrichtung 102 weist in der Umkehrosmoseeinrichtung 118 und/oder der Heizeinrichtung 114 eine Steuerungseinrichtung 116 auf, die ausgebildet ist, den Einlass 110, den Auslass 112 und/oder die Heizeinrichtung 114 parallel oder seriell steuern zu können.

Bei einer Heißdesinfektion der Ringleitung 106 ohne Heißdesinfektion der Umkehrosmoseeinrichtung 118 wird die Heizeinrichtung 114 angeschaltet und das erwärmte Wasser in die Ringleitung 106 geleitet. Das Schaltventil 128 ist dabei geschlossen, um das warme Wasser innerhalb der Heizeinrichtung 114 und der Ringleitung 106 zu halten. Sollte die Heizeinrichtung 114 frisches Wasser benötigen, liefert die Umkehrosmoseeinrichtung 118 dieses nach. Das überschüssige Wasser fließt über das Überströmventil 126 in der Rückführleitung 124 zurück in die Umkehrosmoseeinrichtung 118.

Bei einer Heißdesinfektion der Ringleitung 106 und der Umkehrosmoseeinrichtung 118 wird die Heizeinrichtung 114 angeschaltet und das beheizte Wasser in die Ringleitung 106 geleitet. Das Schaltventil 128 ist dabei geöffnet um das heiße Wasser ebenfalls in die Umkehrosmoseeinrichtung 118 zu leiten. Das Überströmventil 126 ist so eingestellt, dass ein Permeat der Umkehrosmoseeinrichtung 118 durch die Heizeinrichtung 114 fließen muss. Als "Permeat" ist insbesondere Wasser zu verstehen, das mittels einer semipermeablen Membran der Umkehrosmoseeinrichtung 118 von Bestandteilen getrennt wurde, die zuvor in dem Wasser gelöst waren. Das Permeat ist insbesondere dazu geeignet, ohne weitere Behandlung zur Herstellung einer Dialysierflüssigkeit verwendet zu werden.

Auch die Desinfektion der Vorrichtung 102 gemäß der zweiten Ausführungsform ist über eine AO-Wert Berechnung gesteuert. Das heißt, sobald ein vorbestimmter Minimalwert des A0-Werts erreicht wurde, beendet die Vorrichtung 102 die thermische Desinfektion und wechselt in die Abkühlphase, indem warmes Wasser über den Auslass 112 ausgeleitet und durch kälteres Prozesseingangswasser ersetzt wird.

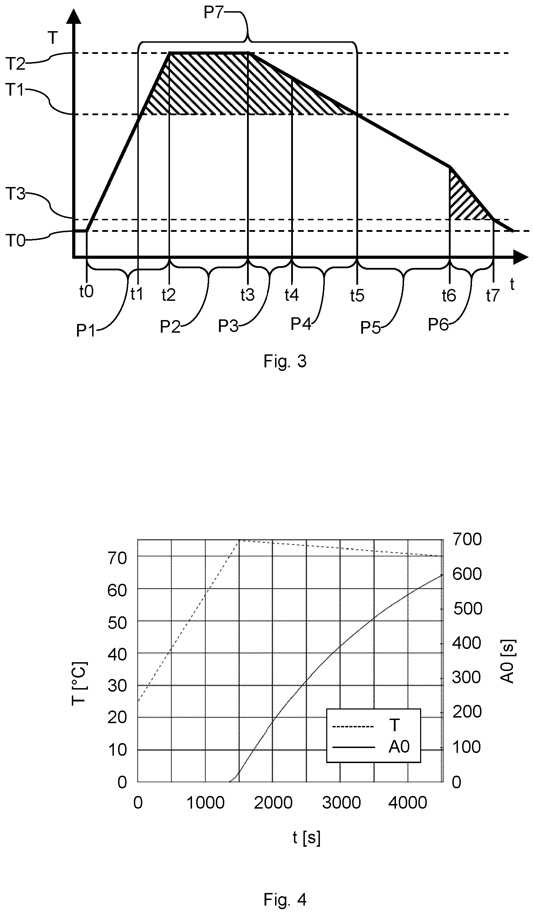

Fig. 3 zeigt ein schematisches Temperatur-Zeit-Diagramm eines offenbarungsgemäßen Verfahrens zur Desinfektion der Vorrichtung 2 bzw. 102 bzw. einen Soll-Verlauf der Fluidleitungssystemtemperatur.

Zu einem Zeitpunkt t0 wird begonnen, das Fluidleitungssystem 4 bzw. 104, das zu Beginn des Desinfektionsverfahrens Umgebungstemperatur T0 aufweist, mittels eines Desinfektionsfluids zu erwärmen.

Zu einem Zeitpunkt t1 erreicht die Fluidleitungstemperatur eine Minimaldesinfektionstemperatur T1, ab welcher Keime in dem Fluidleitungssystem 4 bzw. 104 abgetötet werden.

Die Fluidleitungstemperatur wird nach dem Zeitpunkt t1 weiter erhöht, bis zu einem Zeitpunkt t2 eine maximale Aufheiztemperatur T2 erreicht wird.

Ab dem Zeitpunkt t2 wird die Fluidleitungstemperatur konstant auf dem Wert der maximalen Aufheiztemperatur T2 gehalten, bis die Heizeinrichtung 14 bzw. 114 zu einem Zeitpunkt t3 abgeschaltet wird.

Ab dem Zeitpunkt t3 kühlt das Fluidleitungssystem 4 bzw. 104 passiv, das heißt, ohne Zutun einer Kühleinrichtung, ab.

Zu einem Zeitpunkt t4 erreicht der AO-Wert, das heißt, die in Fig. 3 schraffierte Fläche zwischen dem Soll-Verlauf der Fluidleitungstemperatur und der Isotherme der Minimaldesinfektionstemperatur T1 zwischen t1 und t4, einen Wert, ab welchem das Fluidleitungssystem 4 bzw. 104 als desinfiziert betrachtet werden kann. Um etwaige Unstimmigkeiten während der Desinfektion zu berücksichtigen, ist der Soll-Verlauf der Fluidleitungstemperatur derart festgelegt, dass ein bei einem letztmaligen Unterschreiten der Minimaldesinfektionstemperatur T1 zu einem Zeitpunkt t5 erreichte AO-Wert (der einem offenbarungsgemäßen vorbestimmten Minimalwert einer Soll-Letalität entspricht) größer ist, als der theoretisch notwendige AO-Wert (zum Zeitpunkt t4).

Ab dem Zeitpunkt t5 kühlt das Fluidleitungssystem 4 bzw. 104 weiter passiv ab, bevor zu einem Zeitpunkt t6 eine Aktivkühlung eingeschaltet wird ("Aktivkühlung einschalten" heißt, dass warmes Desinfektionsfluid über den Auslass 12 bzw. 112 aus dem Fluidleitungssystem 4 bzw. 104 ausgelassen wird und kaltes Prozesseingangswasser über den Einlass 10 bzw. 110 in das Fluidleitungssystem 4 bzw. 104 eingelassen wird).

Ab dem Zeitpunkt t6 wird das Fluidleitungssystem 4 bzw. 104 aktiv abgekühlt, so dass die Fluidleitungssystemtemperatur zu einem Einsatzbereitschaftszeitpunkt t7 eine maximale Einsatzbereitschaftstemperatur T3 erreicht. Die maximale Einsatzbereitschaftstemperatur T3 ist im vorliegenden Beispiel höher als die Umgebungstemperatur T0. Alternativ dazu kann auch die Umgebungstemperatur T0 als maximale Einsatzbereitschaftstemperatur T3 definiert werden.

Die Zeitspanne ab einschließlich Zeitpunkt t0 bis ausschließlich Zeitpunkt t2 ist eine Aufheizphase P1. Die Zeitspanne ab einschließlich Zeitpunkt t1 bis ausschließlich Zeitpunkt t3 ist eine Haltephase P2. Die Zeitspanne ab einschließlich Zeitpunkt t3 bis ausschließlich Zeitpunkt t4 ist eine Passivabkühlungsdesinfektionsphase P3. Die Zeitspanne ab einschließlich Zeitpunkt t4 bis ausschließlich Zeitpunkt t5 ist eine Sicherheitsphase P4. Die Zeitspanne ab einschließlich Zeitpunkt t5 bis ausschließlich Zeitpunkt t6 ist eine Passivabkühlungsphase P5. Die Zeitspanne ab einschließlich Zeitpunkt t6 bis ausschließlich Zeitpunkt t7 ist eine Aktivabkühlungsphase P6. Die Zeitspanne ab einschließlich Zeitpunkt t1 bis ausschließlich Zeitpunkt t5 ist eine Desinfektionsphase P7.

Die Steigung des Soll-Verlaufs der Fluidleitungssystemtemperatur in der Aufheizphase P1 entspricht einem offenbarungsgemäßen Aufheizungskoeffizienten. Die Steigung des Soll-Verlaufs der Fluidleitungssystemtemperatur in der Passivabkühlungsdesinfektionsphase P3, der Sicherheitsphase P4 und der Passivabkühlungsphase P5 entspricht einem offenbarungsgemäßen Passivabkühlungskoeffizienten. Die Steigung des Soll-Verlaufs der Fluidleitungssystemtemperatur in der Aktivabkühlungsphase P6 entspricht einem offenbarungsgemäßen Aktivabkühlungskoeffizienten.

Fig. 4 zeigt ein Diagramm, in welchem die Fluidleitungssystemtemperatur und der AO-Wert über die Zeit während eines offenbarungsgemäßen Verfahrens bis zum Erreichen eines vorbestimmten Minimalwerts einer Letalität gezeigt ist. In dem in Fig. 4 gezeigten Beispiel ist die Start- bzw. Umgebungstemperatur T0 gleich 25°C, der vorbestimmte Minimalwert der Soll-Letalität zum Zeitpunkt t5=4500s gleich 600, der Aufheizungskoeffizient 2°C/min und der Passivabkühlungskoeffizient 6°C/h.

Es kann erkannt werden, dass die aktive Heißreinigung lediglich 25.04 Minuten (1502.3 Sekunden) andauert und anschließend in den Standbymodus wechselt. Mit der dadurch erreichten Temperatur von 75.04°C und der angenommenen Abkühlung wird ein AO-Wert von 600 erreicht. In dem in Fig. 4 gezeigten Beispiel, gibt es keine Haltephase.

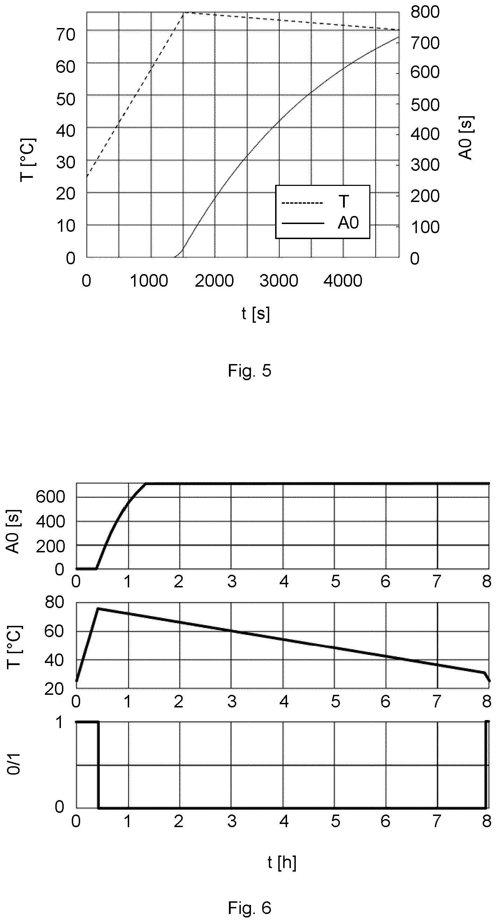

Fig. 5 zeigt ein der Fig. 4 entsprechendes Diagramm zu einem offenbarungsgemäßen Verfahren, bei dem ein erhöhter vorbestimmter Minimalwert der Letalität erreicht wird. Um den gegenüber dem in Fig. 4 gezeigten Beispiel erhöhten vorbestimmten Minimalwert der Letalität 720 zum Zeitpunkt t5=5000s zu erreichen, wird eine höhere maximale Aufheiztemperatur T2 vorgesehen. Da der vorbestimmte Minimalwert der Letalität 720 zu einem späteren Zeitpunkt erreicht wird, als bei dem in Fig. 4 gezeigten Beispiel, ist der Einsatz einer Aktivkühlung in dem in Fig. 5 gezeigten Beispiel eher notwendig.

Fig. 6 zeigt Diagramme, in welchen die Fluidleitungssystemtemperatur, die Letalität bzw. der AO-Wert sowie ein Systemzustand einer medizinischen Vorrichtung 2 bzw. 102 auch nach einem Erreichen des vorbestimmten Minimalwerts der Letalität zu sehen sind.

Wie bei den in den Figuren 4 und 5 gezeigten Beispielen sind keine Haltephasen vorgesehen. Das heißt, sobald die Fluidleitungssystemtemperatur die maximale Aufheiztemperatur T2 erreicht bzw. sobald der Soll-Verlauf der Fluidleitungssystemtemperatur ein Erreichen des Desinfektionszieles zulässt, wird in den gezeigten Beispielen die Heizeinrichtung 14 bzw. 114 ausgeschaltet.

Der vorbestimmte Minimalwert der Letalität wird nach etwa 25 Minuten erreicht (das heißt, t5 = 25 min). Damit die Vorrichtung 2 bzw. 102 zu einem vorbestimmten Einsatzbereitschaftszeitpunkt bereit ist, wird die Vorrichtung ab dem spätest möglichen Zeitpunkt t6 aktiv gekühlt. In dem in Fig. 6 untersten Diagramm ist die Aktivierung der Vorrichtung 2 bzw. 102 gezeigt. Das heißt, in dem untersten Diagramm ist gezeigt, ob die Heizeinrichtung 14 bzw. 114, Pumpen und Ventile der Vorrichtung 2 bzw. 102 abgeschaltet sind oder nicht. In bevorzugter Weise stellt die Vorrichtung 2 bzw. 102 alle Energieverbraucher (außer Steuerungseinheit) ab um Energie während der Passivabkühlung zu sparen.

In einer weiteren vorteilhaften Ausgestaltung können während der passiven Desinfektion/Abkühlungsvorgangs kurzfristig die Pumpen/Ventile geschaltet werden, um das Wasser umzuwälzen. Dies hat das Ziel, eine möglichst homogene Temperaturverteilung im System zu gewährleisten.

In dem in Fig. 6 gezeigten Beispiel muss die Vorrichtung 2 bzw. 102 nur noch 190 Sekunden lang vor dem Einsatzbereitschaftszeitpunkt von ca. 30°C auf 25°C aktiv abgekühlt werden. Durch Berücksichtigung des Einsatzbereitschaftszeitpunkt ist somit eine großes Wasser- und/oder Energieersparnis gegenüber den bekannten Desinfektionsverfahren möglich.

Bezugszeichenliste

| | |
|---|---|
| 2; 102 | Vorrichtung |
| 4; 104 | Fluidleitungssystem |
| 6; 106 | Ringleitung |
| 8; 108 | Temperatursensor |
| 10; 110 | Einlass |
| 12;112 | Auslass |
| 14; 114 | Heizeinrichtung |
| 16; 116 | Steuerungsvorrichtung |
| 118 | Umkehrosmoseeinrichtung |
| 120 | Kühlfluidleitung |
| 122 | Warmfluidleitung |
| 124 | Rückführleitung |
| 126 | Überströmventil |
| 128 | Schaltventil |

| | |
|---|---|
| t0 | Zeitpunkt des Beginns der Aufheizphase |
| t1 | Zeitpunkt des Beginns des Desinfektionsverfahrens |
| t2 | Zeitpunkt des Erreichens der maximalen Aufheiztemperatur |
| t3 | Zeitpunkt des Abschaltens einer Heizeinrichtung |
| t4 | Zeitpunkt des Erreichens eines theoretisch ausreichenden vorbestimmten Minimalwerts einer Letalität |
| t5 | Zeitpunkt des Erreichens eines mit einem Sicherheitsfaktor vorbestimmten Minimalwerts einer Letalität |
| t6 | Zeitpunkt des Einschaltens einer Aktivkühlung |
| t7 | Einsatzbereitschaftszeitpunkt |

| | |
|---|---|
| T0 | Start- bzw. Umgebungstemperatur |
| T1 | Minimaldesinfektionstemperatur |
| T2 | maximale Aufheiztemperatur |
| T3 | maximale Einsatzbereitschaftstemperatur |

| | |
|---|---|
| P1 | Aufheizphase |
| P2 | Haltephase |
| P3 | Passivabkühlungsdesinfektionsphase |
| P4 | Sicherheitsphase |
| P5 | Passivabkühlungsphase |
| P6 | Aktivabkühlungsphase |
| P7 | Desinfektionsphase |

**Patentansprüche**

**1.** Verfahren zum Desinfizieren eines Fluidleitungssystems (4; 104) einer medizinischen Vorrichtung (2; 102), insbesondere einer Blutbehandlungsvorrichtung, vorzugsweise einer Dialysemaschine, oder einer Umkehrosmoseanlage, mit den Schritten:

- Messen einer Fluidleitungssystemtemperatur in einem vorbestimmen Temperaturmessabschnitt innerhalb des Fluidleitungssystems (4; 104),
- Festlegen eines Soll-Verlaufs der Fluidleitungssystemtemperatur mittels eines vorbestimmten Minimalwerts einer Soll-Letalität potentiell in dem Temperaturmessabschnitt vorhandener Keime, wobei die Soll-Letalität mittels Integration des Soll-Verlaufs der Fluidleitungssystemtemperatur über die Zeit ab einem erstmaligen Erreichen einer Minimaldesinfektionstemperatur (T1), ab welcher potentiell in dem Temperaturmessabschnitt vorhandene Keime abgetötet werden können, bis zu einem letztmaligen Unterschreiten der Minimaldesinfektionstemperatur berechnet wird,
- Heizen des Fluidleitungssystems (4; 104) mittels eines Desinfektionsfluids in Abschnitten des Soll-Verlaufs der Fluidleitungssystemtemperatur, in welchen die Fluidleitungssystemtemperatur erhöht oder konstant gehalten werden soll, und
- Abkühlen des Fluidleitungssystems (4; 104) in Abschnitten des Soll-Verlaufs der Fluidleitungssystemtemperatur, in welchen die Fluidleitungssystemtemperatur gesenkt werden soll,

**dadurch gekennzeichnet, dass**

bei dem Festlegen des Soll-Verlaufs der Fluidleitungssystemtemperatur ein Aufheizungskoeffizient, der ein zeitabhängiges Ansteigen der Fluidleitungssystemtemperatur bei einer Aufheizung des Fluidleitungssystems (4; 104) angibt, ein Passivabkühlungskoeffizient, der ein zeitabhängiges Absinken der Fluidleitungssystemtemperatur bei einer Passivabkühlung des Fluidleitungssystems (4; 104) angibt, und ein Einsatzbereitschaftszeitpunkt (t7), zu dem die Fluidleitungssystemtemperatur nach dem letztmaligen Unterschreiten der Minimaldesinfektionstemperatur (T1) kleiner oder gleich einer maximalen Einsatzbereitschaftstemperatur (T3) sein muss, sodass die Vorrichtung (2; 102) nach dem Desinfizieren wieder einsatzbereit ist, berücksichtigt werden und dass vor Erreichen des vorbestimmten Minimalwerts der Soll-Letalität und vor dem letztmaligen Unterschreiten der Minimaldesinfektionstemperatur das Abkühlen des Fluidleitungssystems (4; 104) zumindest zeitweise mittels der Passivabkühlung durch alleiniges Beenden oder Unterbrechen des Heizens erfolgt.

2. Verfahren nach Anspruch 1, **dadurch gekennzeichnet, dass** der Aufheizungskoeffizient und/oder der Passivabkühlungskoeffizient vor dem Festlegen des Soll-Verlaufs der Fluidleitungssystemtemperatur, insbesondere vor oder nach dem Messen der Fluidleitungssystemtemperatur in dem vorbestimmen Temperaturmessabschnitt, mittels zumindest eines Versuchs und/oder mittels einer Simulation festgelegt werden/wird.

3. Verfahren nach Anspruch 1 oder 2, **dadurch gekennzeichnet, dass** der Aufheizungskoeffizient und/oder der Passivabkühlungskoeffizient während einer Durchführung des Verfahrens überwacht werden/wird und bei einer Änderung des Aufheizungskoeffizienten und/oder des Passivabkühlungskoeffizienten der Schritt des Festlegens des Soll-Verlaufs der Fluidleitungssystemtemperatur wiederholt ausgeführt wird.

4. Verfahren nach einem der Ansprüche 1 bis 3, **dadurch gekennzeichnet, dass** bei dem Festlegen des Soll-Verlaufs der Fluidleitungssystemtemperatur ein Aktivabkühlungskoeffizient, der ein zeitabhängiges Absinken der Fluidleitungssystemtemperatur bei einer Aktivabkühlung des Fluidleitungssystems (4; 104) angibt, berücksichtigt wird und das Abkühlen des Fluidleitungssystems (4; 104) zeitweise mittels der Aktivabkühlung durch Abführen warmen Desinfektionsfluids und/oder Zuführen eines Kühlmittels erfolgt.

5. Verfahren nach Anspruch 4, **dadurch gekennzeichnet, dass** der Aktivabkühlungskoeffizient vor dem Festlegen des Soll-Verlaufs der Fluidleitungssystemtemperatur, insbesondere vor oder nach dem Messen der Fluidleitungssystemtemperatur in dem vorbestimmen Temperaturmessabschnitt, mittels zumindest eines Versuchs und/oder mittels einer Simulation festgelegt wird.

6. Verfahren nach Anspruch 4 oder 5, **dadurch gekennzeichnet, dass** der Aktivabkühlungskoeffizient während einer Durchführung des Verfahrens überwacht wird und bei einer Änderung des Aktivabkühlungskoeffizienten der Schritt des Festlegens des Soll-Verlaufs der Fluidleitungssystemtemperatur wiederholt ausgeführt wird.

7. Verfahren nach einem der Ansprüche 4 bis 6, **dadurch gekennzeichnet, dass** mittels der vorbestimmten Soll-Letalität, des Einsatzbereitschaftszeitpunkts (t7), des Passivabkühlungskoeffizienten und des Aktivabkühlungskoeffizienten ein letztmöglicher Aktivabkühlungsstartzeitpunkt (t6) berechnet wird, nach welchem die maximale Einsatzbereitschaftstemperatur (T3) nur noch mittels der Aktivkühlung erreicht werden kann und bis zu welchem das Fluidleitungssystem (4; 104) ausschließlich mittels der Passivkühlung gekühlt wird.

8. Verfahren nach einem der Ansprüche 1 bis 7, **dadurch gekennzeichnet, dass** bei einer Festlegung des vorbestimmten Minimalwerts der Soll-Letalität ein Sicherheitsfaktor einberechnet wird.

9. Verfahren nach einem der Ansprüche 1 bis 8, **dadurch gekennzeichnet, dass** der Soll-Verlauf der Fluidleitungssystemtemperatur derart festgelegt wird, dass ein mittleres Niveau der Fluidleitungssystemtemperatur zwischen dem erstmaligen Erreichen der Minimaldesinfektionstemperatur (T1) und dem letztmaligen Unterschreiten der Minimaldesinfektionstemperatur (T1) über der Minimaldesinfektionstemperatur (T1) liegt.

10. Verfahren nach Anspruch 9, **dadurch gekennzeichnet, dass** das mittlere Niveau der Fluidleitungssystemtemperatur zwischen dem erstmaligen Erreichen der Minimaldesinfektionstemperatur (T1) und dem letztmaligen Unterschreiten der Minimaldesinfektionstemperatur (T1) in Richtung der Minimaldesinfektionstemperatur abgesenkt wird, wenn erkannt wird, dass die Fluidleitungssystemtemperatur nach dem letztmaligen Unterschreiten der Minimaldesinfektionstemperatur (T1) ohne der Aktivkühlung vor dem Einsatzbereitschaftszeitpunkt (t7) auf die maximale Einsatzbereitschaftstemperatur (T3) abgesenkt werden kann.

11. Medizinische Vorrichtung (2; 102), insbesondere eine Blutbehandlungsvorrichtung, vorzugsweise eine Dialysemaschine, oder eine Umkehrosmoseanlage, mit

einem Fluidleitungssystem (4; 104),
zumindest einem Temperatursensor (8; 108), der ausgebildet ist, eine Fluidleitungssystemtemperatur in einem vorbestimmen Temperaturmessabschnitt innerhalb des Fluidleitungssystems (4; 104) messen zu können,
einem Einlass (10; 110) zum Einleiten eines Desinfektionsfluids und/oder eines Kühlmittels in das Fluidleitungssystem (4; 104),
einem Auslass (12; 112) zum Ausleiten des Desinfektionsfluids und/oder des Kühlmittels aus dem Fluidleitungssystem (4; 104),
einer Heizeinrichtung (14; 114) zum Heizen des Desinfektionsfluids und einer Steuerungseinrichtung (16; 116) zum Steuern des Einlasses (10; 110), des Auslasses (12; 112) und der Heizeinrichtung (14; 114) in Abhängigkeit der von dem zumindest einen Temperatursensor (8; 108) gemessenen Fluidleitungssystemtemperatur,

**dadurch gekennzeichnet, dass**

die Steuerungseinrichtung (16; 116) ausgebildet ist, auf eine Anforderung hin ein Verfahren gemäß einem der Ansprüche 1 bis 10 auszuführen.

Fig. 1

Fig. 2

Fig. 3

Fig. 4

Fig. 5

Fig. 6